# EUROPÄISCHE PATENTSCHRIFT

(11) **EP 0 572 992 B1**
(45) Veröffentlichungstag und Bekanntmachung des Hinweises auf die Patenterteilung: **20.08.2003**
(21) Anmeldenummer: 93108830.6
(22) Anmeldetag: 02.06.1993
(51) Int. Cl.: C12N 9/54, C11D 3/386

(54) **Alkalische Proteasen aus Bacillus pumilus**
Alkaline proteases from Bacillus pumillus
Protéases alcalines de Bacillus pumillus

(30) Priorität: 04.06.1992 DE 4218448
(43) Veröffentlichungstag der Anmeldung: 08.12.1993
(73) Patentinhaber: GENENCOR INTERNATIONAL, INC., Palo Alto, California 94304 (US)
(72) Erfinder: Vetter, Roman, Dr., W-3167 Burgdorf (DE); Wilke, Detlef, Dr., W-3015 Wennigsen (DE); Möller, Bernhard, Dr., W-5110 Alsdorf (DE); Lerch, Martina, D-30926 Seelze (DE); Mücke, Ingo, Dr., W-3013 Barsinghausen (DE); Takenberg, Meike, W-3000 Hannover 81 (DE); Konieczny-Janda, Gerhard, W-3017 Pattensen (DE)
(74) Vertreter: Kiddle, Simon John

(56) Entgegenhaltungen:
- BIOSIS PREVIEWS DATABASE,Philadelphia Ab.Number: 92009219 QIU X; DAI H; YUAN Y; YU Y 'STUDIES ON ALKALINE...'
- BIOSIS PREVIEWS DATABASE,Philadelphia Ab.Number: 92009219 QIU X; DAI H; YUAN Y; YU Y 'STUDIES ON ALKALINE...'
- BIOSIS PREVIEWS DATABASE,Philadelphia Ab.Number: 62060151 'FERMENTATIVE PRODUCTION...'
- DATABASE WPI Week 8617, Derwent Publications Ltd., London, GB; AN 86-112132 Ä17Ü
- BIOSIS PREVIEWS DATABASE,Philadelphia Ab. Number: 91015259 QIU X; YUAN Y; DAI H; YU Y 'STUDY ON ALKALINE...'
- DATABASE WPI Week 8922, Derwent Publications Ltd., London, GB; AN 89-160751 Ä22Ü
- ZEITSCHRIFT F]R ALGEMEINE MIKROBIOLOGIE Bd. 21, Nr. 7, 1981, BERLIN DE Seiten 531 - 536 Okotore RO; Akinrimisi EO; Ojo MO 'A novel extracellular proteinase from Bacillus pumilus.'
- DATABASE WPI Week 9142, Derwent Publications Ltd., London, GB; AN 91-307004 (42)

## Beschreibung

Die vorliegende Erfindung bezieht sich auf alkalische Proteasen aus Bacillus pumilus, deren Verwendung und ein Verfahren zur Herstellung dieser Proteasen.

Alkalische Proteasen sind wertvolle industrielle Produkte mit vorteilhaften Anwendungen, insbesondere in der Waschmittelindustrie, da sie proteinenthaltende Verunreinigungen entfernen. Um wirksam zu sein, müssen diese Proteasen nicht nur proteolytische Aktivität unter Waschbedingungen (pH-Wert, Temperatur) besitzen, sondern sie müssen darüber hinaus auch mit anderen Waschmittelbestandteilen, d.h. in Kombination mit anderen Enzymen, Tensiden, Gerüststoffen (Builder), Bleichmitteln, Bleichmittelaktivatoren und anderen Zusatz- und Hilfsstoffen verträglich sein. Insbesondere müssen die Proteasen gegenüber diesen Waschmittelbestandteilen ausreichende Stabilität und in deren Gegenwart ausreichende Waschwirksamkeit aufweisen.

Die alkalischen Proteasen des Standes der Technik werden bisher insbesondere durch Kultivierung von Bacillus-Spezies wie z.B. Bacillus alcalophilus, Bacillus subtilis, Bacillus amyloliquefaciens und Bacillus licheniformis, die alkalische Proteasen produzieren und in das Kulturmedium ausscheiden, erhalten.

In Bezug auf diese alkalischen Proteasen wurden im Stand der Technik zwar bereits viele Anstrengungen unternommen, um neue alkalische Proteasen mit gewünschten Eigenschaften zu erhalten. So sind bereits eine Reihe von natürlichen und künstlich (gentechnisch) veränderten alkalischen und hochalkalischen Proteasen bekannt. Dennoch besteht Bedarf an neuen, alkalischen Proteasen mit insbesondere im Hinblick auf das Waschverhalten güngstigen Eigenschaften.

Acta Micrbiol. Sin. 15(4):330-334, 1976 veröffentlicht die Gärung von *Bacillus pumilus* Stamm 209, und dass die Enzymbrühe eine Tätigkeit von 5.000 Einheiten/ml und ein optimales pH zwischen 9 und 11 und eine optimale Temperatur von 50°C hatte.

Es bestand daher die Aufgabe, neue wertvolle alkalische Proteasen mit günstigen Eigenschaften herzustellen.

Die erfindungsgemäßen Proteasen sind durch Kultivierung von Bacillus pumilus DSM 5777 oder durch Kultivierung eines transformierten Mikroorganismus Bacillus, welcher die genetische Information für eine der erfindungsgemäßen Proteasen aus Bacillus pumilus DSM 5777, in einem Expressionsvektor, enthält, erhältlich.

Eine Auftrennung des Kulturüberstandes einer Kultur von Bacillus pumilus DSM 5777 mit Hilfe der HPLC (= High pressure liquid chromatography) zeigt, daß aus Bacillus pumilus DSM 5777 zumindest zwei Proteasen mit unterschiedlichen Eigenschaften erhältich sind.

Eine der erfindungsgemäßen Proteasen aus Bacillus pumilus DSM 5777 ist eine isolierte alkalische Bacillus-Protease mit den folgenden Eigenschaften:
(1) Wirkung: Abbau von Proteinen und Peptiden;
(2) pH-Optimum: etwa bei pH-Werten von 10,5 bis 11,0;
(3) pH-Stabilität: bei pH-Werten von 9,7 bis 10,8 erweisen sich die Enzyme als völlig stabil;
(4) Temperatur-Optimum: etwa 60 °C,
(5) Temperatur-Stabilität: durch Inkubation der Protease bei Temperaturen bis 45 °C für 15 Minuten wird die Protease nicht wesentlich in ihrer Aktivität beeinträchtigt; nach 15 Minuten Inkubation bei etwa 50 °C beträgt die Restaktivität der Protease wenigstens 90 %.

Die andere der erfindungsgemäßen isolierten, alkalischen Proteasen aus Bacillus pumilus DSM 5777 besitzt die folgenden Eigenschaften:
(1) Wirkung: Abbau von Proteinen und Peptiden;
(2) pH-Optimum: etwa bei pH-Werten von 8,5 bis 9;
(3) pH-Stabilität: bei pH-Werten von 5,5 bis 10,5 erweisen sich die Enzyme als völlig stabil;
(4) Temperatur-Optimum: etwa 50 °C;
(5) Temperatur-Stabilität: durch Inkubation der Protease bei Temperaturen bis 40 °C für 15 Minuten wird die Protease nicht wesentlich in ihrer Aktivität beeinträchtigt; nach 15 Minuten Inkubation bei etwa 45 °C beträgt die Restaktivität der Protease wenigstens 95 %.

Die erfindungsgemäßen Bacillus-Proteasen eignen sich als Additive für Waschmittel- und Reinigungsmittelzusammensetzungen etc., die neutrale bis alkalische pH-Werte besitzen und bei niedrigen Temperaturen, insbesondere bei Temperaturen bis 60 °C angewendet werden sollen. Die Erfindung betrifft daher auch die Verwendung der erfindungsgemäßen alkalischen Bacillus-Proteasen in Waschmittel-, Reinigungsmittel- oder Geschirrspülmittelzusammensetzungen. Sie können hierbei auch vorteilhaft in Gegenwart von anderen üblichen Enzymen, insbesondere auch in Gegenwart von anderen Proteasen, eingesetzt werden. Eine besonders bevorzugte Verwendung der erfindungsgemäßen alkalischen Proteasen betrifft deren Verwendung in Waschmittel-, Reinigungsmittel- oder Geschirrspülmittelzusammensetzungen für niedrige Anwendungstemperaturen, insbesondere bis etwa 60 °C, vorzugsweise für Anwendungstemperaturen von etwa 30 bis 60 °C.

Weiterhin umfaßt die Erfindung Waschmittel-, Reinigungsmittel- und Geschirrspülmittelzusammensetzungen, die wenigstens eine der erfindungsgemäßen alkalischen Proteasen enthalten. Für diese Anwendungszwecke stellt die Erfindung eine Gruppe neuer alkalischer Proteasen mit günstigen Eigenschaften zur Verfügung mit denen sich vorteilhaft proteinenthaltende Verschmutzungen entfernen lassen. Sowohl Eigelb als auch Blut und Milch enthaltende Verschmutzungen werden in gleich guter Weise beseitigt. Eine Beeinträchtigung durch die übrigen in den Waschmittelformulierungen enthaltenden Bestandteilen ist in der Waschwirksamkeit der erfindungsgemäßen Proteasen praktisch nicht festzustellen.

Die erfindungsgemäßen Proteasen können in Wasch- und Reinigungsmittelformulierungen, beispielsweise in Pulverwaschmittelformulierungen, einzeln oder gewünschtenfalls auch in Kombination miteinander, gegebenenfalls auch in Kombination mit Wasch- und Reinigungsmittelproteasen des Standes der Technik oder anderen in solchen Zusammensetzungen üblichen Enzymen, wie z.B. Proteasen, Amylasen, Lipasen, Pektinasen, Nukleasen, Oxidoreduktasen, Cellulasen etc., eingesetzt werden. Die erfindungsgemäßen Proteasen werden in den Wasch- und Reinigungsmittelformulierungen in an sich für Waschmittelenzyme üblichen Mengen, insbesondere in Mengen von bis zu 3 Gew.-% (bezogen auf die Trockensubstanz der Gesamtzusammensetzung), vorzugsweise in einer Menge von 0,2 bis 1,5 Gew.-%, verwendet.

Außer den bereits erwähnten Waschmittelenzymen können die Wasch- und Reinigungsmittel der Erfindung alle an sich im Stand der Technik üblichen Waschmittelinhaltsstoffe wie Tenside, Bleichmittel oder Gerüststoffe (Builder), sowie weitere übliche Hilfsstoffe für die Formulierung von Waschmitteln in an sich üblichen Mengen enthalten. Zu den Hilfsstoffen gehören z.B. Verstärker, Enzymstabilisatoren, Schmutzträger und/oder Kompatibilisierungsmittel, Komplexund Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen.

So enthalten erfindungsgemäße Waschmittelformulierungen in typischer beispielhafter Zusammensetzung bezogen auf Trockensubstanz
a) wenigstens 5 Gew.-%, z.B. 10 bis 50 Gew.-%, eines Tensids oder Tensidgemisches
b) bis zu 40 Gew.-% eines Builders oder eines Builder-Gemisches,
c) bis zu 40 Gew.-% eines Bleichmittels oder Bleichmittelgemisches, vorzugsweise ein Perborat wie Natriumperborat-Tetrahydrat oder Natriumperborat-Monohydrat,
d) bis zu 3 Gew.-% wenigstens einer erfindungsgemäßen Protease
e) weitere Bestandteile wie Hilfsstoffe etc. ad 100 Gew.-%

Solche Waschmittelformulierungen können in an sich üblicher Weise formuliert werden. Die erfindungsgemäßen Proteasen können dazu z.B. in Form von Granulaten, Prills oder Pellets, gegebenenfalls auch mit Oberflächenüberzügen versehen, mit den anderen Komponenten der Waschmittelformulierung in an sich bekannter Weise vermischt werden.

Die erfindungsgemäßen Proteasen eignen sich darüber hinaus sehr gut für den Einsatz in an sich üblichen Flüssigwaschmittelformulierungen.

Die erfindungsgemäßen alkalischen Proteasen können erhalten werden, indem man Bacillus pumilus DSM 5777 oder einen in einem Expressionsvektor die genetische Information für eine der erfindungsgemäßen Proteasen enthaltenden Mikroorganismus kultiviert und nachfolgend aus dem Kulturüberstand die gebildete alkalische Protease isoliert. Die Isolierung der alkalischen Proteasen aus dem Kulturüberstand wird dabei in an sich bekannter Weise durchgeführt, indem man die Zellen durch Filtration oder durch Zentrifugation abtrennt, die Protease durch Membranfiltration oder Fällung konzentriert, aufgereinigt, ggf. auch isoliert und einer gewünschten Verwendung zuführt.

Für die Herstellung und Gewinnung der erfindungsgemäßen Proteasen können z.B. der Bacillus pumilus-Stamm DSM 5777 selbst oder ein transformierter Bacillus pumilus-Stamm, der die genetische Information für eine der erfindungsgemäßen Proteasen, in einem Expressionsvektor, enthält, verwendet werden.

Für die großtechnische Produktion, insbesondere aus Gründen der Produktionsvereinfachung und -optimierung sowie zur Ausbeutesteigerung, können auch andere Mikroorganismen, insbesondere Bacillus-Stämme, in die zuvor durch Transformation die notwendige genetische Information über die erfindungsgemäßen Proteasen und deren Expression eingebracht wurde, zur Herstellung und Gewinnung der erfindungsgemäßen Proteasen eingesetzt werden.

Die Erfindung umfaßt daher auch ein Verfahren zur Herstellung der erfindungsgemäßen alkalischen Proteasen mit transformierten Mikroorganismen, welche einen Expressionsvektor mit zur Protease-Expression benötigten DNA-Sequenzen und mit einer solchen DNA-Sequenz enthalten, welcher für eine Aminosäurensequenz einer der vorstehend beschriebenen erfindungsgemäßen, alkalischen Protease codiert. Der erfindungsgemäß transformierte Mikroorganismus wird wie oben angegeben kultiviert und aus dem Kulturmedium die alkalische Protease isoliert. Bevorzugte transformierte Mikroorganismen für die Herstellung und Gewinnung der erfindungsgemäßen Protease sind Bacillus-Spezies wie Bacillus subtilis, Bacillus alcalophilus, Bacillus licheniformis oder Bacillus amyloliquefaciens. Die erfindungsgemäß transformierten Mikroorganismen zeichnen sich insbesondere dadurch aus, daß sie mit einem Expressionsyektor transformiert sind, der die genetische Information für eine erfindungsdemäße alkalische Protease aus Bacillus pumilus enthält, der am 09.02.1990 unter der Nummer DSM 5777 bei der Deutschen Sammlung von Mikroorganismen, Bundesrepublik Deutschland, hinterlegt worden ist. Ebenfalls umfaßt die Erfindung daher auch die am 23.02.1992 unter den Nummern DSM 6879 und DSM 6880 hinterlegten Mikroorganismen sowie die aus diesen Mikroorganismen isolierbaren Plasmide, die die für die vorstehend beschriebenen erfindungsgemäßen Proteasen genetischen Informationen enthalten.

Zur Erzeugung der im vorstehenden Verfahren eingesetzten, transformierten Mikroorganismen, kann so vorgegangen werden, daß man
a) zunächst aus einem geeigneten Bakterien-Stamm, welcher eine alkalische Protease mit einer Aminosäurensequenz mit mindestens 70 %, vorzugsweise über 80 %, insbesondere aber über 90 %, Homologie zu der Aminosäurensequenz der Protease aus Bacillus pumilus DSM 5777 produziert, die für die Protease codierende DNA-Sequenz (d.h. das Strukturgen der Protease) isoliert,
b) ggf. die Nukleotidabfolge dieser DNA-Sequenz zur weiteren Identifizierung der Protease bestimmt,
c) nachfolgend mit Hilfe der isolierten DNA-Sequenz einen Expressionsvektor herstellt und
d) den erhaltenen Expressionsvektor in einen geeigneten Mikroorganismus, welcher schließlich zur Produktion der alkalischen Protease eingesetzt werden kann, transformiert.

Die Verfahrensschritte zur Isolierung und Gewinnung der erfindungsgemäßen alkalischen Proteasen nach dem vorstehenden Verfahren, sowie die hierbei erhaltenen, Zwischenprodukte in Form von DNA-Sequenzen bzw. DNA-Inserts mit dem Protease-Gen, Vektoren, insbesondere Expressionsvektoren, und transformierten Mikroorganismen werden nachfolgend im einzelnen näher beschrieben.

Die Strukturgene, die für Aminosäurensequenzen der erfindungsgemäßen alkalischen Proteasen codieren, können nach an sich bekannten, allgemeinen Methoden erhalten werden. Hierzu wird z.B. aus Bacillus pumilus DSM 5777 ("Donor-Bacillus ") die chromosomale DNA nach an sich bekannten Methoden isoliert und mit geeigneten Restriktionsendonukleasen partiell hydrolysiert.

Die erhaltenen Restriktionsfragmente der Donor-DNA können durch Gelelektrophorese oder Zentrifugation durch einen Saccharose-Dichte-Gradienten nach Größe aufgetrennt und die Fragmente gewünschter Größe dann mit einer geeigneten, Vektor-DNA rekombiniert werden. Zweckmäßig wird als Vektor ein Plasmid verwendet, mit dem die Expression der in den verwendeten Wirtsorganismus eingebrachten Fremd-DNA möglich ist. Wie in den Beispielen beschrieben, kann z.B. ein Plasmid mit der Bezeichnung pUB131, das aus dem Plasmid pUB110 durch Einführung einer Polylinkerstelle erzeugt wurde, verwendet werden.

Die vorstehend erhaltene, in vitro rekombinierte DNA kann nun in geeignete Wirtszellen, z.B. in den hier verwendeten Stamm Bacillus subtilis PSL 1 eingebracht werden. Transformanten, d.h. Wirtszellen, die die rekombinante DNA aufgenommen haben, können mit Hilfe von bekannten Markern auf der Vektor-DNA (z.B. Neomycin-Resistenz) selektiert werden. Unter diesen antibiotikumresistenten Transformanten kann nach solchen Klonen, die vermehrt Protease ausscheiden, gesucht werden. Unter derartigen Transformanten können solche isoliert werden, die in der Lage sind, eine erfindungsgemäße Protease auszuscheiden. Aus einem positiven Klon wird schließlich die in diese Transformante eingeführte Plasmid-DNA isoliert.

Dieses Plasmid enthält neben der Vektor-DNA mit bekannten Restriktionsstellen das gewünschte Strukturgen für die erfindungsgemäße alkalische Protease aus Bacillus pumilus DSM 5777 und gegebenenfalls weitere, hier aber nicht benötigte DNA-Sequenzen aus dem Donor-Bacillus. Beispiele für solche Protease-haltigen Plasmide sind die Plasmide mit der Bezeichnung pPP46 und pPP415. Das Plasmid pPP46 hat eine Größe von 7,6 Kilobasen und codiert für die erfindungsgemäße Protease P46 aus Bacillus pumilus DSM 5777. Das Plasmid pPP415 hat eine Größe von 6,2 Kilobasen und codiert für die erfindungsgemäße Protease P415 aus Bacillus pumilus DSM 5777.

Die Fähigkeit dieser Plasmide zur Expression der entsprechenden alkalischen Protease kann überprüft werden, indem man ein Bakterium, insbesondere eine Bacillus-Spezies der oben genannten Art, mit einem dieser Plasmide transformiert und die so erhaltenen Transformanten kultiviert und auf Proteaseaktivität überprüft. Die erhaltenen Transformanten können darüber hinaus auch zur Herstellung und Gewinnung der erfindungsgemäßen alkalischen Proteasen kultiviert werden, wobei dann die weiter oben beschriebenen erfindungsgemäßen alkalischen Proteasen erhalten werden.

Die erfindungsgemäßen Proteasen aus Bacillus pumilus DSM 5777 zeichnen sich durch vorteilhafte Eigenschaften aus. Sie besitzen bei alkalischen pH-Werten eine hohe Stabilität. Das pH-Optimum der erfindungsgemäßen Proteasen liegt in einem für die Anwendung in Wasch- und Reinigungsmittelzusammensetzungen günstigen Bereich von etwa pH 8,0 bis 11,5. Ferner besitzen die erfindungsgemäßen Proteasen ein Temperatur-Optimum im Bereich von etwa 50 bis 60 °C. Ferner zeigen sie gute Stabilitäten auch in Waschlauge. Aufgrund ihrer günstigen Aktivität bei Temperaturen bis zu 60 °C eignen sich die erfindungsgemäßen Bacillus-Proteasen insbesondere für die Anwendung in Reinigungs- und Waschmittelzusammensetzungen, die bei niedrigen Temperaturen, insbesondere bis 60 °C, vorzugsweise zwischen 30 und 60 °C, angewendet werden sollen. Solche Wasch- und Reinigungsmittelzusammensetzungen, die eine erfindungsgemäße Protease enthalten, zeigen günstige Waschwirksamkeit hinsichtlich zu entfernender Proteinanschmutzungen.

Die nachfolgenden Beispiele offenbaren die Erfindung in typischen beispielhaften Ausgestaltung, ohne jedoch die Erfindung in ihrem Umfange zu beschränken.

Sofern nicht anders angegeben, wurde generell nach Methoden gearbeitet, wie sie in Maniatis et al. (Maniatis et al. = T. Maniatis, E. F. Fritsch, J. Sambrook, "Molecular Cloning, A Laboratory Manual", Cold Spring Harbor Laboratory, 1982) beschrieben sind.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktions-, Cofaktor- und übrigen Bedingungen sind ebenfalls bekannt. Z.B. kann-für eine Menge von etwa 1 µg DNA eine Einheit (= U = Unit) der Restriktionsendonuklease in etwa 20 µl einer Pufferlösung eingesetzt werden. Ausreichende Inkubationszeiten von etwa einer Stunde bei 37 °C wurden gewöhnlich eingehalten, die Inkubationsbedingungen können aber den gegebenen Erfordernissen angepaßt werden. Nach Inkubation mit einer Restriktionsendonuklease wurde das Protein durch Extraktion (z.B. mit Phenol und Chloroform) entfernt und die geschnittene DNA (z.B. aus der wäßrigen Fraktion durch Fällung mit Ethanol) isoliert und der weiteren Verwendung zugeführt.

An das Schneiden von DNA oder Vektoren mit Restriktionsendonukleasen kann sich gegebenenfalls eine Hydrolyse des terminalen 5'-Phosphatrestes mit einer alkalischen Phosphatase (Dephosphorylierung) anschließen. Sofern in den Beispielen eine Dephosphorylierung des 5'-Endes vorgenommen wurde, geschah dieses in an sich bekannter Weise. Weitere Angaben zur Durchführung einer Dephosphorylierung und zu dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und das gewünschte DNA-Fragment aus der entsprechenden Gelzone abgetrennt.

Ligationen können unter an sich bekannten Bedingungen, z.B. in einem Puffer mit etwa 10 Units T4-DNA-Ligase pro 0,5 µg der zu ligierenden DNA-Fragmente, ausgeführt werden. (siehe z.B. Maniatis et al., S. 146).

Unter Transformation wird die Einschleusung von DNA in einen Mikroorganismus verstanden, so daß die DNA in diesem repliziert bzw. exprimiert werden kann. Für Bacillus-Spezies ist z.B. die Methode nach Anagnostopolous et al. (1961, J. Bacteriol. 81: 746 - 791) geeignet.

Bei der Angabe von Enzymstabilitäten in den folgenden Beispielen bedeuten: "völlig stabil" eine Restaktivität von mindestens 90 %; "stabil" eine Restaktivität von wenigstens 80 %.

Der hier verwendete, im Beispiel 1 und der Beschreibung angegebene Bacillus pumilus-Stamm wurde aus der Natur isoliert und bei der Deutschen Sammlung von Mikroorganismen (DSM) unter der Nummer DSM 5777 am 07.02.1990 hinterlegt. Der im Beispiel 3 angegebene mit dem Plasmid pPP46 transformierte Stamm B. subtilis PSL1 wurde unter der Nummer DSM 6879 und der im Beispiel 3 angegebene mit dem Plasmid pPP415 transformierte Stamm B. subtilis PSL1 unter der Nummer DSM 6880 bei der Deutschen Sammlung für Mikroorganismen am 22.01.1992 hinterlegt. Andere verwendete Mikroorganismen, z.B. Bacillus subtilis PSL1 (Bacillus Genetic Stock Center 1 A 510) oder Bacillus BD366 (Bacillus Genetic Stock Center E 6) sind käuflich verfügbar.

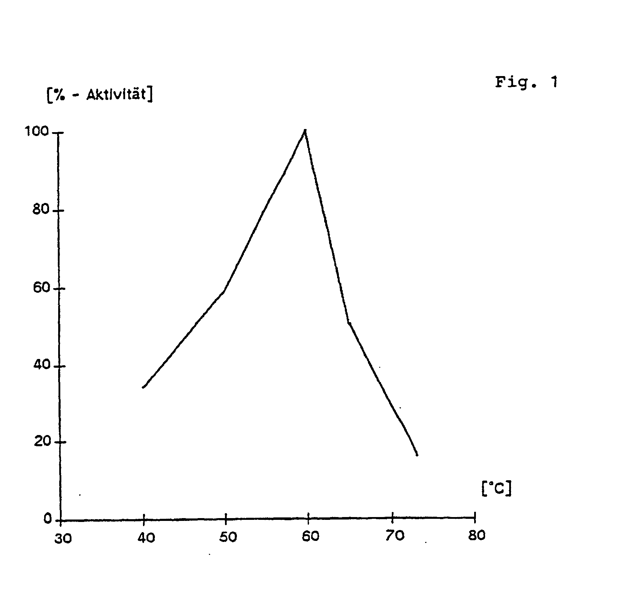

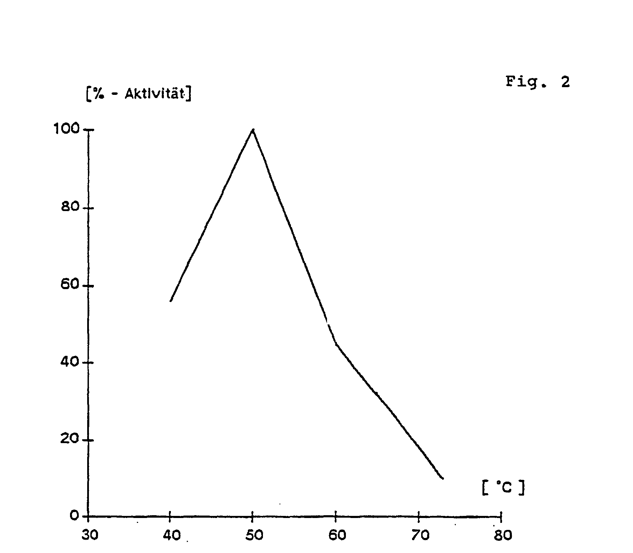

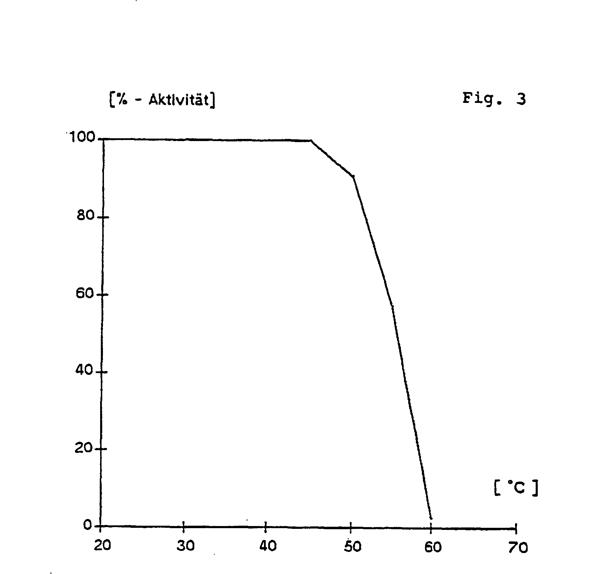

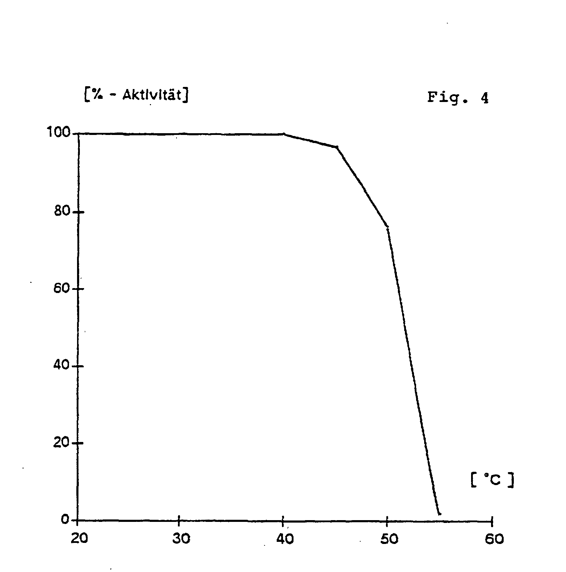

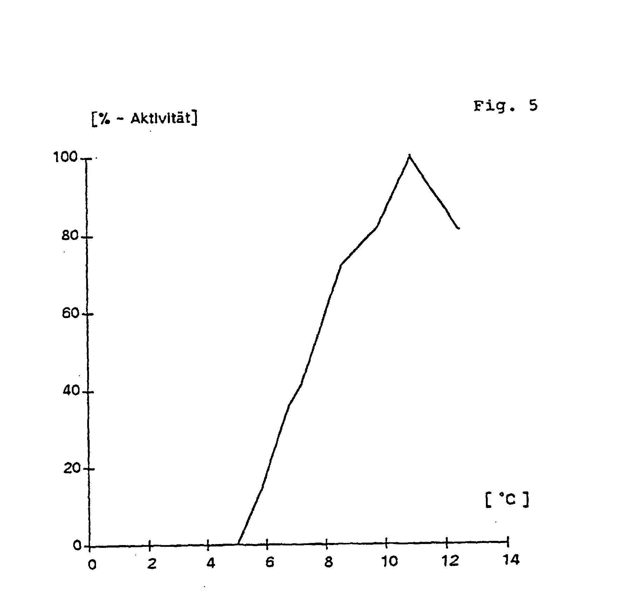

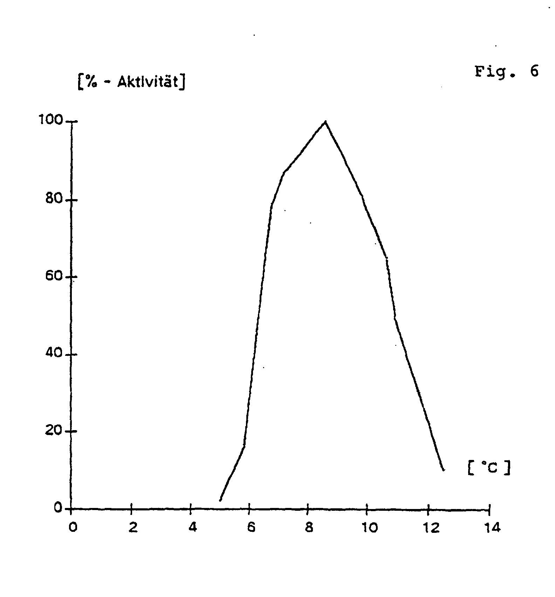

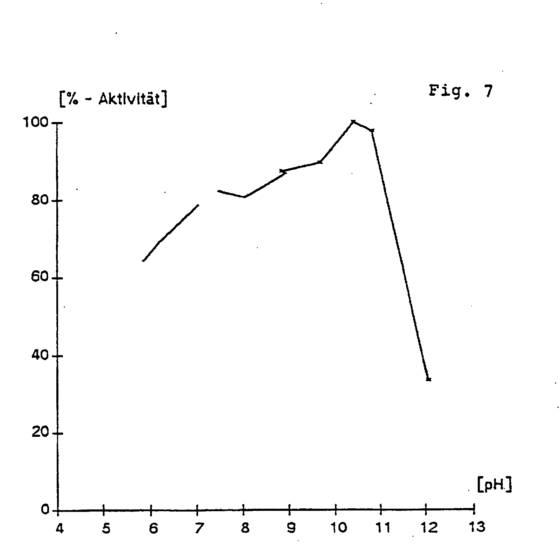

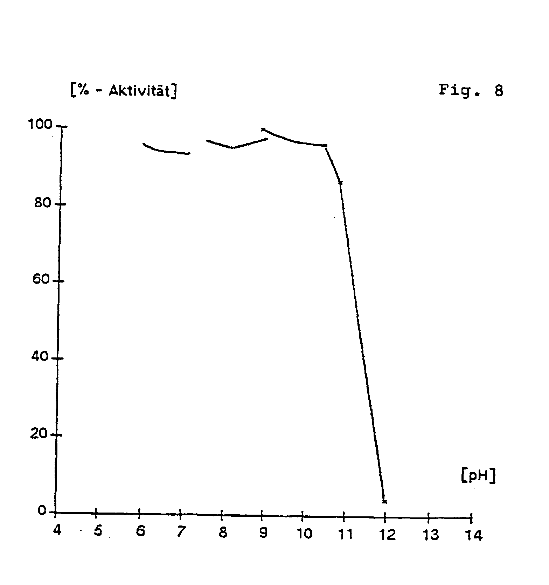

Erläuterungen zu den Figuren:
Figur 1 und 2:
   Temperatur-Optimum der Proteasen P46 (Fig. 1) und P415 (Fig.2) aus Bacillus pumilus DSM 5777.
Figur 3 und 4:
   Temperatur-Stabilität der Proteasen P46 (Fig. 3) und P415 (Fig. 4) aus Bacillus pumilus DSM 5777.
Figur 5 und 6:
   pH-Optimum der Proteasen P46 (Fig. 5) und P415 (Fig. 6) aus Bacillus pumilus DSM 5777.
Figur 7 und 8:
   pH-Stabilität der Proteasen P46 (Fig. 7) und P415 (Fig. 8) aus Bacillus pumilus DSM 5777. Zur pH-Wert-Einstellung wurde für den pH-Bereich pH 5 bis pH 7 0,1 M Phosphatpuffer, für den Bereich pH 7 bis pH 9 0,1 M Tris-HCl-Puffer und für den Bereich pH 9 bis pH 12,1 0,2 M Glycin-NaOH-Puffer benutzt.
Figur 9:
   Waschwirksamkeit der Protease P415 auf Testgewebe M-PC in Abhängigkeit von der Enzym-Dosierung (delta R = Reflektionsdifferenz).
Figur 10:
   Waschwirksamkeit der Protease P415 auf Testgewebe Y-MC in Abhängigkeit von der Enzym-Dosierung (delta R = Reflektionsdifferenz).
Figur 11:
   Waschwirksamkeit der Protease P415 auf Testgeweben in Abhängigkeit von der Enzym-Dosierung (delta R = Reflektionsdifferenz).

### Beispiel 1

### Isolierung eines Bacillus pumilus aus der Natur und dessen Identifizierung.

Der Stamm Bacillus pumilus wurde aus der Natur isoliert und bei der Deutschen Sammlung von Mikroorganismen unter der Nummer DSM 5777 hinterlegt.

Es handelt sich um einen grampositiven, sporenbildenden aeroben Mikroorganismus der Gattung Bacillus. Die zell- und koloniemorphologische Beschreibung wird im folgenden gegeben, biochemische Reaktionen und Reaktionen auf bestimmte Wuchsbedingungen sind in der Tabelle 1 aufgeführt.

### Bacillus pumilus DSM 5777:

Stäbchenförmiges Bakterium mit abgerundeten Ecken. Die Gramreaktion (Gramfärbung, KOH-Test) ist positiv. Auf TY-Agar (siehe unten) haben die Kolonien nach 2 Tagen bei 37 °C einen Durchmesser von 3,2 bis 4,2 mm, sind beigefarben und haben einen glatten bis wellenförmigen Rand. Gelegentlich ist auf den Kolonien auch Tröpfchenbildung zu beobachten; die Kolonien können glänzend oder-eingetrocknet-runzlig sein. Die Zellen haben auf TY-Agar eine Größe von 0,8 bis 0,9 µm * 1,2 bis 2,8 µm und liegen in der Regel als einzelne Zellen oder in Zweier- oder Dreierketten vor. Die Sporen sind oval und zentral bis subterminal gelegen. Der Stamm sporuliert bereitwillig.

| TY-Agar: | |
|---|---|
| Hefeextrakt | 5 g |
| MgCL₂ *6H₂O | 8,75 g |
| MnCl₂*2H₂O | 0,016 g |
| Agar | 16 g |
| Aqua bidest. | ad 1000 ml |
| pH | 7,0 ± 0,3 |

Aufgrund der nach Durchführung der in der Tabelle 1 genannten Tests (nach Bergeys Manual of Systematic Bacteriology, Vol.2, 1121-1125, P.H.A. Sneath (ed.), Williams und Wilkins, Baltimore-London-Los Angeles-Sydney, 1986) gefundenen Resultate kann eine Zuordnung zu der Art Bacillus pumilus vorgenommen werden. Der isolierte Stamm DSM 5777 weicht lediglich in wenigen Merkmalen (Voges-Proskauer Test, pH der V-P-Nährlösung und Bildung von Eigelb-Lecithinase, sowie hinsichtlich des Wachstums bei 7 % NaCl) von den dort für Bacillus pumilus aufgeführten Kennzeichen ab.

Bacillus pumilus ist ein ubiquitär vorkommender Organismus, der auf Nährböden Kolonien mit variablem Aussehen bildet.

**Tabelle 1***

| | Bacillus | |
|---|---|---|
| Merkmal | DSM 5777 | pumilus |
| Zelldurchmesser > 1µm | - | - |
| Sporenform rund | - | - |
| Sporangium geschwollen | - | - |
| Parasporale Kristalle | - | - |
| Catalase | + | + |
| Anaerobes Wachstum | - | *-* |
| Voges-Proskauer Test | - | + |
| pH in V-P Nährlösung | | |
| < 6 | | + |
| > 7 | + | |
| Säurebildung auf | | |
| Glucose | + | + |
| L-Arabinose | + | + |
| D-Xylose | + | + |
| D-Mannitol | + | + |
| Gasbildung mit Glucose | - | - |
| Hydrolyse von | | |
| Casein | + | + |
| Gelatine | + | + |
| Stärke | - | - |
| Metabolisierung von | | |
| Citrat | + | + |
| Propionat | - | - |
| Abbau von Tyrosin | - | - |
| Desaminierung von Phenylalanin | - | - |
| Eigelb-Lecithinase | + | - |
| Nitratreduktion zu Nitrit | - | - |
| Bildung von | | |
| Indol | - | - |
| Dihydroxyaceton | - | n.b. |
| NaCl- u. KCl-Bedürfnis | n.b. | - |
| Allantoin- oder Ureat-Bedürfnis | n.b. | - |
| Wachstum bei pH = | | |
| 6,8 in NB | + | + |
| 5,7 in NB | + | + |
| Wachstum in NaCl | | |
| 2 % | n.b. | + |
| 5 % | + | + |
| 7 % | - | + |
| 10 % | - | n.b. |
| Wachstum bei | | |
| 5 °C | n.b. | - |
| 10 °C | n.b. | + |
| 30 °C | + | + |
| 40 °C | n.b. | + |
| 50 °C | n.b. | d |
| 55 °C | n.b. | - |
| 65 °C | n.b. | - |
| Wachstum in Gegenwart von Lysozyme | + | d |
| Autotroph mit H₂ + CO₂ oder CO | n.b. | - |
| * Angaben von B. pumilus nach Bergey's Manual of Systematic Bacteriology, Vol. 2, S. 1123, P.H.A. Sneath, ed., Williams u. Wilkins, Baltimore-Lond Los Angeles-Sydney, 1986. +, 90 % oder mehr positiv -, 90 % oder mehr negativ d, 11-89 % positiv n.b., nicht bestimmt NB, Nährbouillon | | |

### Beispiel 2

### Herstellung des Plasmids pUB131.

Aus dem Stamm Bacillus subtilis BD366 (pUB110) (Bacillus Genetic Stock Center 1 E 6) wurde nach der Methode von T.J. Gryczan et al. (1978, J.Bacteriol. 34:318-329) das Plasmid pUB110 isoliert und anschließend nach Maniatis et al. (S. 93) über Cäsiumchlorid-Dichtegradientenzentrifugation gereinigt. Der Vektor pUB110 enthält nur einmal vorkommende Restriktionsstellen für die Restriktionsendonukleasen BamHI und EcoRI und als Marker eine DNA-Sequenz, die für Antibiotikaresistenz gegenüber Neomycin codiert, sowie für die Replikation in Bacillus-Spezies benötigte DNA-Sequenzen ("origin of replication").

Das vorstehend erhaltene Plasmid pUB110 wurde mit EcoRI und BamHI restringiert, wobei ein größeres und ein kleineres Fragment erhalten wurde. Das kleinere Fragment 790 bp (bp = Basenpaare) wurde ersetzt durch einen 67 bp großen Polylinker, der zuvor als EcoRI/Bg1II-Fragment aus dem Vektor M13tg131 isoliert worden war. Der so erhaltene Vektor mit der Bezeichnung pUB131 ist somit ein Abkömmling von pUB110, bei dem das etwa 0,8 KB große EcoRI/BamHI-Fragment deletiert und dafür eine Polyklonierungsstelle eingebaut wurde.

### Beispiel 3

### Klonierung der alkalischen Proteasen P46 und P415 aus Bacillus pumilus DSM 5777.

Aus dem Naturisolat Bacillus pumilus DSM 5777 des Beispiels 1 wurde nach der Methode von Saito et al. (1963, Biochim. Biophys. Acta. 72, Seiten 619 - 629) die chromosomale DNA isoliert und mit der Restriktionsendonuklease Sau3A partiell hydrolisiert. Die Restriktionsfragmente wurden durch Elektrophorese auf einem Agarosegel aufgetrennt und die Fragmente mit einer Größe von 3 bis 8 kB (kB = Kilobasen) wurden isoliert.

Die isolierten DNA-Fragmente aus dem Bacillus DSM 5777 wurden mit Vektor-DNA des Plasmids pUB131 (Herstellung wie in Beispiel 3 beschrieben) in vitro neukombiniert.

Hierzu wurde das Plasmid pUB131 zunächst mit der Restriktionsendonuklease BamHI restringiert und anschließend mit alkalischer Phosphatase aus Kälberdarm dephosphoryliert. Anschließend wurden 4 µg der restringierten und dephosphorylierten Vektor-DNA mit 20 µg der DNA-Fragmente aus dem Bacillus DSM 5777 in einem Gesamtvolumen von 200 µl mit T4-DNA Ligase 24 h bei 16 °C inkubiert.

Mit der in vitro neukombinierten DNA wurden Protoplasten des Stammes Bacillus subtilis PSL1 (Bacillus Genetic Stock Center 1 A 510) nach der von S. Chang und N. Cohen (1979, Mol. Gen. Genet. 168, Seiten 111 - 115) beschriebenen Methode transformiert. Die Transformanten wurden auf Platten mit Neomycin selektiert und anschließend auf Magermilchagar-Platten überführt. Unter ca. 100.000 erhaltenen neo^{r}-Transformanten wurden einige Klone gefunden, die aufgrund klarer Höfe um die jeweilige Kolonie als verstärkte Proteaseausscheider identifiziert werden konnten.

Diese Klone wurden in TY-Medium mit 10 µg/ml Neomycin (10 g Trypton, 5 g Hefeextrakt, 4,1 mg MgCl₂ x 2H₂O und 16 mg MnCl₂ x 2H₂O ad 1000 ml Aqua bidest.) für 24 h kultiviert. Die Proteasen in den Kulturüberständen wurden auf einer HPLC-Säule - Ultropak TSK CM-2SW, 250 x 4,6 mm, (Firma: LKB) - getrennt und mit UV-Licht (280 mm) detektiert. Eine angekoppelte Fließinjektionsanalyse (Substrat: Acetylcasein; Detektionsreagenz: Trinitrobenzolsulfonsäure; Detektion bei 420 nm) gab die Möglichkeit, die Peaks mit proteolytischer Aktivität von anderen Proteinpeaks zu differenzieren. Neben Klonen, die nur die wirtseigenen Proteasen verstärkt ausschieden, wurden solche gefunden, die neben den wirtseigenen Proteasen jeweils auch eine wirtsfremde Protease ausschieden. Bezüglich der Elutionszeiten konnten zwei unterschiedlich wirtsfremde Proteasen identifiziert werden, welche die Bezeichnungen Protease P46 und P415 erhielten. Die Plasmide aus diesen Klonen, die die Proteasen, die im Kulturüberstand von Bacillus pumilus DSM 5777 zu finden sind, ausschieden und somit die genetische Information dieser Proteasen enthalten, erhielten die Bezeichungen pPP46 beziehungsweise pPP415. Das Plasmid pPP46 hat eine Größe von 7,6 kB und das Plasmid pPP415 eine Größe von 6,2 kB. Der mit dem Plasmid pPP46 transformierte Stamm B. subtilis PSL1 wurde am 23.01.1992 bei der Deutschen Sammlung für Mikroorganismen unter der Nummer DSM 6879 hinterlegt. Der mit dem Plasmid pPP415 transformierte Stamm B. subtilis PSL1 wurde am 23.01.1992 bei der Deutschen Sammlung für Mikroorganismen unter der Nummer DSM 6880 hinterlegt.

### Beispiel 4

### Herstellung der alkalischen Proteasen P46 und P415 aus B. pumilus DSM 5777.

Die Plasmide pPP46 und pPP415 wurden aus den Klonen B. subtilis PSL1 (pPP46) und B. subtilis PSL1 (pPP415) des Beispiels 3 isoliert und in den Donorstamm B. pumilus DSM 5777 eingebracht. Die Transformation erfolgte als Protoplastentransformation nach der Methode von Chang und Cohen (siehe Beispiel 2). Anschließend wurden 40 ml Vorkulturmedium (10 g Tryptone, 5 g Hefe-Extrakt, 5 g NaCl, 10 mg Neomycin, Aqua bidest. ad 1000 ml) in 500 ml Schüttelkolben mit einer Einzelkolonie des Stammes B. pumilus DSM 5777 (pPP46) oder des Stammes B. pumilus DSM 5777 (pPP415) von einer Agarplatte beimpft. Die Kultur wurde für 16 h bei 37 °C und 250 Upm inkubiert. Mit 1 ml dieser Kultur wurden 40 ml Hauptkulturmedium (40 g Sojamehl, 90 g Kartoffelstärke, 1,5 g Na₂SO₄, 3,5 mg MnCl₂, 10 mg Neomycin und Aqua bidest. ad 1000 ml) in 500 ml Schüttelkolben beimpft. Die Hauptkultur wurde für 30 h und 320 Upm unter den ansonsten gleichen Bedingungen wie die Vorkultur inkubiert und nach 30 h zentrifugiert.

Aus dem Überstand konnten die alkalischen Proteasen über FPLC (CM Sepharose FF (Pharmacia) Elutionspuffer 0,05 M Na-Acetat pH 6 und 0,8 M Na-Acetat pH 6) gereinigt werden oder der Kulturüberstand direkt für weitere Versuche verwendet werden.

### Beispiel 5

### Bestimmung der Enzymcharakteristika von Bacilllus pumilus-Proteasen.

Die Aktivität der Proteasen wurde in Delft Units (DU) bestimmt. 1000 DU ist die proteolytische Aktivität, die bei einem Volumen von 1 ml einer 2 % (w/w)igen Enzymlösung nach Abbau von Casein eine Extinktionsdifferenz (1 cm Lichtweg; 275 nm; Bestimmung gegen Blindprobentest) von 0,4000 ergibt.

Zur Bestimmung der Enzymcharakteristika wie Temperatur-Optimum, Temperatur-Stabilität, pH-Optimum und pH-Stabilität wurden die im Beispiel 4 durch Kultivierung der Stämme Bacillus pumilus DSM 5777 (pPP46) und DSM 5777 (pPP415) erhaltenen Kulturüberstände verwendet.

Das Temperatur-Optimum der in den Kulturüberständen enthaltenen Proteasen wurde im Bereich von 40 bis 73 °C bestimmt. Die Ergebnisse sind in Tabelle 2 sowie in den Figuren 1 bzw. 2 dargestellt.

Das Temperatur-Optimum der Protease P46 aus Bacillus pumilus DSM 5777 (pPP46) liegt bei 60 °C (Fig. 1).

Das Temperatur-Optimum der Protease P415 aus Bacillus pumilus DSM 5777 (pPP415) liegt bei 50 °C (Fig. 2).

**Tabelle 2**

| Temperatur-Optimum der alkalischen Proteasen aus Bacillus pumilus DSM 5777 (pPP46) und DSM 5777 (pPP415) | | | | | |
|---|---|---|---|---|---|
| alkalische Protease | Aktivität in % in Abhängigkeit von der Temperatur in °C | | | | |
| | 40 °C | 50 °C | 60 °C | 65 °C | 73 °C |
| P46 | 34 % | 59 % | 100 % | 50 % | 16 % |
| P415 | 56 % | 100 % | 45 % | 32 % | 10 % |

Zur Bestimmung der Temperatur-Stabilität wurden die proteasehaltigen Überstände für 15 Minuten bei verschiedenen Temperaturen inkubiert und anschließend die Restaktivität bestimmt. Die Ergebnisse sind in der Tabelle 3 sowie in den Figuren 3 und 4 dargestellt.

Die Protease P46 aus Bacillus pumilus DSM 5777 (pPP46) ist bis 50 °C stabil (Restaktivität > 90 %) und zeigt nach 15-minütiger Inkubation bei 55 °C noch eine Restaktivität von 57,3 % (Fig. 3).

Die Protease P415 aus Bacillus pumilus DSM 5777 (pPP415) ist bis 45 °C stabil (Restaktivität > 90 %) und zeigt nach 15-minütiger Inkubation bei 50 °C noch eine Restaktivität von 76,4 % (Fig. 4).

**Tabelle 3**

| Temperatur [°C] | proteolytische Restaktivität in % nach 15-minütiger Inkubation bei verschiedenen Temperaturen | |
|---|---|---|
| | P46 | P415 |
| 20 | 100 | 100 |
| 30 | 100 | 100 |
| 40 | 100 | 100 |
| 45 | 100 | 96, 9 |
| 50 | 90, 9 | 76,4 |
| 55 | 57,3 | 1,9 |
| 60 | 2,3 | <0,2 |
| 65 | <0,2 | <0,2 |

Zur Bestimmung der pH-Optima der Bacillus-Proteasen wurde die Aktivität bei verschiedenen pH-Werten bestimmt. Für die Einstellung der pH-Werte wurde im pH-Bereich 5 bis 7 Phosphat- (0,1 M), im pH-Bereich 7,0 bis 9,0 Tris-HCl-(0,1 M) und im pH-Bereich 9,0 bis 13,0 Glycin-NaOH-Puffer (0,1 M) verwendet. Die Werte der Aktivitätsbestimmung sind in Tabelle 4 und in den Abbildungen 5 und 6 dargestellt.

Das pH-Optimum der alkalischen Protease P46 aus Bacillus pumilus DSM 5777 (pPP46) liegt bei pH 10,9. Bei pH 12,5 ist die Aktivität noch > 80 % (Fig. 5).

Das pH-Optimum der alkalischen Protease P415 aus Bacillus pumilus DSM 5777 (pPP415) liegt bei etwa pH 8,6 (Fig. 6).

**Tabelle 4**

| alkalische Protease | Proteaseaktivität in % in Abhängigkeit vom pH-Wert | | | | | | | | |
|---|---|---|---|---|---|---|---|---|---|
| | pH | | | | | | | | |
| | 5,0 | 5,8 | 6,8 | 7,2 | 8,6 | 9,8 | 10,6 | 10,9 | 12,5 |
| P46 | 0 | 14 | 36 | 41 | 72 | 82 | 95 | 100 | 81 |
| P415 | 2 | 16 | 79 | 87 | 100 | 81 | 65 | 50 | 10 |

Zur Untersuchung der pH-Stabilität wurden die Proteasen für 24 Stunden in Puffern verschiedenen pH-Wertes bei 4 °C inkubiert. Anschließend wurde die Restaktivität der Proteasen bestimmt. Für den pH-Bereich von 5 bis 7,1 wurde Phosphat-Puffer (0,1 M), für den pH-Bereich von 7,1 bis 9 Tris-HCL-Pfuffer (Tris (hydroxymethyl) aminomethan-Puffer), (0,1 M) und für den pH-Bereich von 9 bis 12,1 Glycin/Natriumhydroxid-Puffer (0,1 M) verwendet. Die Ergebnisse sind in den Figuren 7 und 8 dargestellt.

Die Protease P46 aus Bacillus pumilus DSM 5777 (pPP46) ist zwischen pH 5,8 bis 9,7 weitgehend und zwischen pH 9,7 bis 10,8 völlig stabil; nach 24 Stunden bei pH 12,06 sind noch 34 % Restaktivität nachweisbar (Fig. 7).

Die Protease P415 Bacillus pumilus DSM 5777 (pPP415) ist zwischen pH 5,8 bis 10,4 völlig und zwischen pH 10,4 bis 10,8 weitgehend stabil (Fig. 8).

### Beispiel 6

### Waschleistungen der Protease P46.

Die Wirkung der erfindungsgemäßen Protease P46 wurde in Waschversuchen ermittelt.

### Beispiel 6a

Für die Anwendung als Additiv in Waschmitteln wurde die Waschleistung der erfindungsgemäßen Protease durch Waschversuche am Testgewebe EY-PC (Eigelb/Tusche-Anschmutzung auf Polyester-Baumwolle-Mischgewebe - eigene Herstellung) in Laborwaschmaschinen (Typ: Polycolor) bestimmt. Hierzu wurde das Testgewebe mit 6 g/l einer Waschmittelbasisformulierung, die 18,4 % Zeolith, 7,3 % Na₂CO₃, 4,8 % Lineares Alkylbenzolsulfonat, 3,3 % Nonionics, 3,3 % Seife, 0,1 % Entschäumer, 1,5 % Carboxymethylcellulose, 0,15 % optischen Aufheller, 3,5 % Natrium-di-Silikat, 25 % Perborat, 1,5 % TAED und 30,85 % Na₂SO₄ enthielt, nach Zusatz der erfindungsgemäßen Protease (5.000 DU/l) mit Wasser von 15 °dH gewaschen. Der pH-Wert der Waschlauge betrug pH 10,3. Gewaschen wurde im Temperaturbereich von 15 °C bis 60 °C für 45 min (2 °C/min; 22,5 min Haltezeit).

Die enzymhaltige Waschmittellösung wirkte in einem rotierenden Probengefäß, das über ein Wasserbad entsprechend dem Temperaturprogramm gesteuert wurde, auf das Testgewebe ein. Nach dem Waschvorgang wurde das Testgewebe zweimal mit Leitungswasser gespült und anschließend gebügelt.

Die Waschwirksamkeit wurde durch Messung der Reflektion des gewaschenen Testgewebes bestimmt. Die Waschwirksamkeit, die durch das Enzym bewirkt wird, ergibt sich aus dem höheren Reflektionsvermögen des mit dem enzymhaltigen Waschmittel gewaschenen Testgewebes. Bei nur mit der Waschmittelbasisformulierung gewaschenen Testgewebe wurde für die Reflektion des Testgewebes ein Wert von 41 bestimmt. Bei Zusatz der erfindungsgemäßen Protease P46 zur Waschmittelbasisformulierung wurde für die Reflektion des Testgewebes mit 45 ein deutlich höherer Wert ermittelt.

### Beispiel 6b

Die Untersuchung der Waschwirksamkeit der erfindungsgemäßen Protease P46 wurde wie in Beispiel 6a beschrieben durchgeführt. Die Waschmittelbasisformulierung war ein handelsübliches Pulvervollwaschmittel (pH 10,4). Die Bestimmung der Reflektion des Testgewebes für die Waschmittelbasisformulierung ergab einen Wert von 41. Bei Zusatz der erfindungsgemäßen Protease P46 zur Waschmittelbasisformulierung wurde mit einem Wert von 48 eine deutlich höhere Reflektion des Testgwebes gemessen.

### Beispiel 6c

Die Untersuchung der Waschwirksamkeit der erfindungsgemäßen Protease P46 wurde wie in Beispiel 6b beschrieben durchgeführt. Es wurde jedoch als Testgewebe ein mit Blut, Milch und Tusche angeschmutztes Polyester-Baumwolle-Mischgewebe (EMPA 117 - bezogen von der Eidgenössischen Materialprüfungsanstalt, ST. Gallen, Schweiz) verwendet. Als Waschmittelbasisformulierung wurden 4 g/l eines handelsüblichen Flüssigwaschmittels (pH 7,5) eingesetzt. Gewaschen wurde im Temperaturbereich von 15 °C bis 40 °C (2 °C/min; 22,5 min Haltezeit).

Ohne Zusatz der erfindungsgemäßen Protease P46 wurde für die Reflektion des gewaschenen Testgewebes ein Wert von 50, bei Zusatz der erfindungsgemäßen Protease P46 wurde für die Reflektion des gewaschenen Testgewebes ein Wert von 62 ermittelt.

### Beispiel 6d

Die Untersuchung der Waschwirksamkeit der erfindungsgemäßen Protease P46 wurde wie in Beispiel 6c beschrieben durchgeführt. Es wurde jedoch als Testgewebe ein mit Eigelb und Tusche angeschmutztes Polyester-Baumwolle-Mischgewebe verwendet.

Ohne Zusatz der Protease P46 wurde ein Reflektionswert des gewaschenen Testgewebes von 41, bei Zusatz der erfindungsgemäßen Protease P46 zur Waschmittelbasisformulierung ein Wert von 45 gemessen.

### Beispiel 6e

Die Untersuchung der Waschwirksamkeit der erfindungsgemäßen Protease P46 wurde wie in Beispiel 6d beschrieben durchgeführt. Es wurde jedoch als Testgewebe ein mit Milch und Tusche angeschmutztes Polyester-Baumwolle-Mischgewebe verwendet.

Ohne Zusatz der Protease P46 wurde für die Reflektion des gewaschenen Testgewebes ein Wert von 45, bei Zusatz der erfindungsgemäßen Protease P46 zur Waschmittelbasisformulierung ein Wert von 52 gemessen.

Die Ergebnisse der Beispiele 6a bis 6e belegen die hervorragenden Waschleistungen der erfindungsemäßen Protease P46. Das Reflektionsvermögen des gewaschenen Testgewebes ist bei Zusatz der erfindungsgemäßen Protease zur Waschmittelbasisformulierung deutlich höher, was ein Indiz für das gute Waschvermögen der erfindungsgemäßen Protease P46 ist. Proteinverunreinigungen unterschiedlicher Art werden in hervorragender Weise durch die Protease P46 aus dem Gewebe entfernt.

### Beispiel 7

### Waschleistungen der Protease P415.

Die Untersuchung der Waschwirksamkeit der erfindungsgemäßen Protease P415 wurde wie in Beispiel 6 für die Protease P46 beschrieben, durchgeführt.

### Beispiel 7a

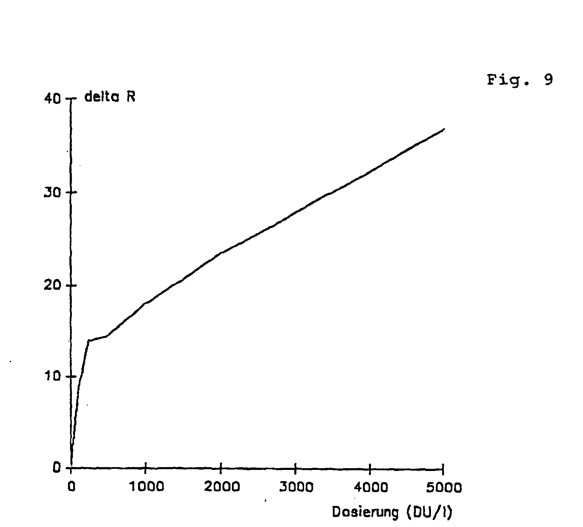

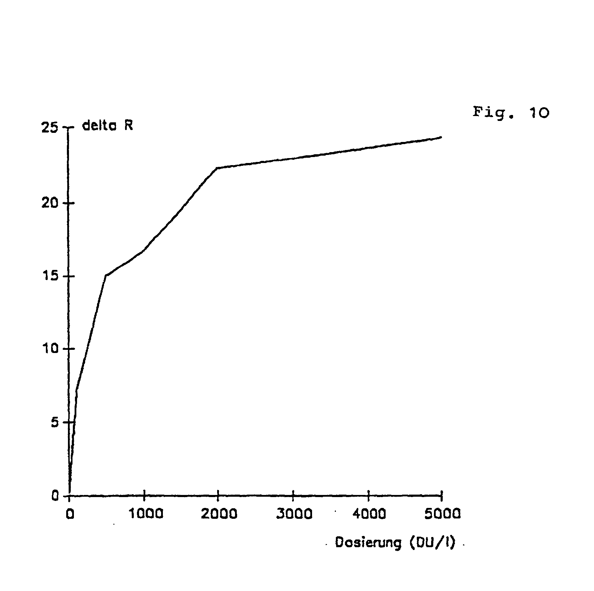

Es wurde die Waschwirksamkeit in Abhängigkeit von der Enzym-Dosierung (DU/ml) auf zwei verschiedenen Testgeweben (M-PC = Milch/Tusche-Anschmutzung auf Polyester-Baumwolle-Mischgewebe; EY-PC = Eigelb/Tusche-Anschmutzung auf Polyester-Baumwolle-Mischgewebe) untersucht. Als Waschmittelbasisformulierung wurde ein handelsübliches europäisches Flüssigwaschmittel verwendet. Die Bestimmung der Reflektions des gewaschenen Testgewebes erfolgte, wie in Beispiel 6 beschrieben. Die Ergebnisse der Waschversuche sind in Figur 9 (M-PC) und Figur 10 (EY-PC) wiedergegeben, in denen die Reflektionsdifferenz des unter Zusatz der erfindungsgemäßen Protease P415 gewaschenen Testgewebes zu dem ohne Zusatz der Protease P415 gewaschenen Testgewebe in Abhängigkeit von der Enzym-Dosierung aufgetragen ist.

### Beispiel 7b

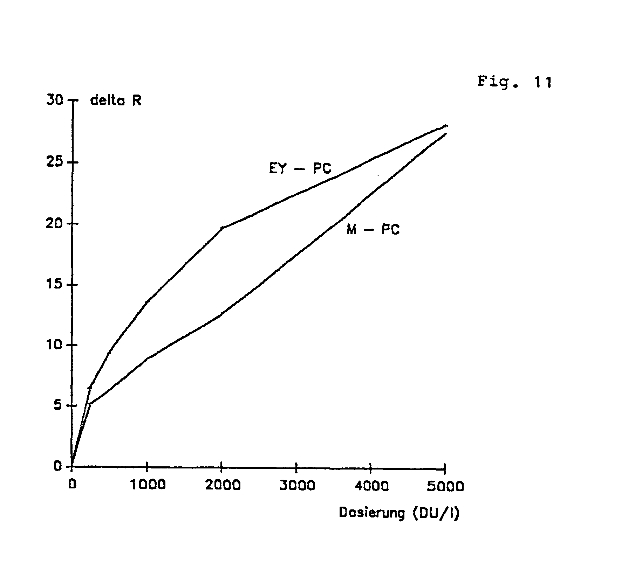

Die Untersuchung der Waschwirksamkeit der erfindungsgemäßen Protease P415 wurde wie in Beispiel 7a beschrieben durchgeführt. Es wurde jedoch als Waschmittelbasisformulierung ein handelsübliches europäisches Kompaktwaschmittel (pH 10,3) verwendet. Die Dosierung betrug 3 g/l. Die Ergebnisse der Waschversuche sind in Figur 11 als Reflektionsdifferenzen in Abhängigkeit von der Enzym-Dosierung wiedergegeben.

Die Ergebnisse der Beispiele 7a und 7b zeigen die hervorragende Waschwirksamkeit der erfindungsgemäßen Protease P415, wobei auch in geringer Dosierung sehr gute Waschleistungen möglich sind. Proteinverunreinigungen unterschiedlicher Art werden in hervorragender Weise durch die Protease P415 beim Waschvorgang aus dem Gewebe entfernt.

## Patentansprüche

1. Isolierte alkalische Bacillus-Protease, erhältlich durch Kultivierung von Bacillus pumilus DSM 5777, mit folgenden Eigenschaften:
(1) Wirkung: Abbau von Proteinen und Peptiden;
(2) pH-Optimum: etwa bei pH-Werten von 10,5 bis 11,0
(3) pH-Stabilität: bei pH-Werten von 9,7 bis 10,8 erweisen sich die Enzyme als völlig stabil;
(4) Temperatur-Optimum: etwa 60 °C;
(5) Temperatur-Stabilität: durch Inkubation der Protease bei Temperaturen bis 45 °C für 15 Minuten wird die Protease nicht wesentlich in ihrer Aktivität beeinträchtigt; nach 15 Minuten Inkubation bei 50 °C beträgt die Restaktivität der Protease wenigstens 90 %.

2. Isolierte alkalische Bacillus-Protease, erhältlich durch Kultivierung von Bacillus pumilus DSM 5777, mit folgenden Eigenschaften:
(1) Wirkung: Abbau von Proteinen und Peptiden;
(2) pH-Optimum: etwa bei pH-Werten von 8,5 bis 9;
(3) pH-Stabilität: bei pH-Werten von 5,5 bis 10,5 erweisen sich die Enzyme als völlig stabil;
(4) Temperatur-Optimum: etwa 50 °C;
(5) Temperatur-Stabilität: durch Inkubation der Protease bei Temperaturen bis 40 °C für 15 Minuten wird die Protease nicht wesentlich in ihrer Aktivität beeinträchtigt; nach 15 Minuten Inkubation bei 45 °C beträgt die Restaktivität der Protease wenigstens 95 %.

3. Zusammensetzungen zum Waschen, Reinigen oder Geschirrspülen, enthaltend eine alkalische Bacillus-Protease gemäß einem der Ansprüche 1 oder 2.

4. Zusammensetzungen nach Anspruch 3, **dadurch gekennzeichnet, daß** sie die alkalische Bacillus-Protease in Gegenwart eines oder mehrerer von in Wasch- und Reinigungsmitteln üblichen Enzymen aus der Gruppe der Proteasen, Lipasen, Pektinasen, Amylasen, Nukleasen, Oxidoreduktasen und Cellulasen enthalten.

5. Zusammensetzungen nach Anspruch 4, **dadurch gekennzeichnet, daß** sie die alkalische Bacillus-Protease in Gegenwart einer zweiten Protease enthalten.

6. Zusammensetzungen nach Anspruch 5, **dadurch gekennzeichnet, daß** sie eine Kombination aus einer alkalischen Bacillus-Protease gemäß Anspruch 1 und einer alkalischen Bacillus-Protease gemäß Anspruch 2 enthalten.

7. Zusammensetzungen nach einem der Ansprüche 3 bis 6, **dadurch gekennzeichnet, daß** sie die alkalische Bacillus-Protease in einer Formulierung für niedrige Anwendungstemperaturen bis etwa 60 °C enthalten.

8. Verfahren zur Herstellung von alkalischen Bacillus-Proteasen gemäß einem der Ansprüche 1 oder 2, **dadurch gekennzeichnet, daß** man den Bacillus pumilus DSM 777 oder einen in einem Expressionsvektor die genetische Information für eine der in den Ansprüchen 1 bis 2 angegebenen Proteasen enthaltenden Mikroorgansimus kultiviert und nachfolgend aus dem Kulturüberstand die gebildete alkali-sche Protease isoliert.

9. Verfahren nach Anspruch 8, **dadurch gekennzeichnet, daß** man als Mikroorganismus, einen Bacillus subtilis oder einen Bacillus alcalophilus oder einen Bacillus licheniformis oder einen Bacillus amyloliquefaciens oder einen Bacillus pumilus einsetzt.

10. Verfahren nach Anspruch 9, **dadurch gekennzeichnet, daß** der Expressionsvektor ein aus dem unter der Nummer DSM 6879 hinterlegten Mikroorganismus oder ein aus dem unter der Nummer DSM 6880 hinterlegten Mikroorganismus isolierbares Plasmid ist.

11. Unter der Nummer DSM 6879 und der Nummer DSM 6880 hinterlegte Mikroorganismen.

12. Aus dem unter der Nummer DSM 6879 hinterlegten Mikroorganismus isolierbares, die genetische Information für die im Anspruch 1 angegebene Protease enthaltendes Plasmid.

13. Aus dem unter der Nummer DSM 6880 hinterlegten Mikroorganismus isolierbares, die genetische Information für die im Anspruch 2 angegebene Protease enthaltendes Plasmid.

## Claims

1. Isolated alkaline Bacillus protease obtainable by cultivation of Bacillus pumilus DSM 5777 and having the following properties:
(1) action: breakdown of proteins and peptides;
(2) pH optimum: approximately at pH values from 10.5 to 11.0
(3) pH stability: at pH values from 9.7 to 10.8 the enzymes prove to be completely stable;
(4) temperature optimum: about 60°C;
(5) thermal stability: incubation of the protease at temperatures up to 45°C for 15 minutes does not substantially impair the protease in its activity; the retained activity of the protease is at least 90% after 15 minutes of incubation at 50°C.

2. Isolated alkaline Bacillus protease obtainable by cultivation of Bacillus pumilus DSM 5777 and having the following properties:
(1) action: breakdown of proteins and peptides;
(2) pH optimum: approximately at pH values from 8.5 to 9;
(3) pH stability: at pH values from 5.5 to 10.5 the enzymes prove to be completely stable;
(4) temperature optimum: about 50°C;
(5) thermal stability: incubation of the protease at temperatures up to 40°C for 15 minutes does not substantially impair the protease in its activity; the retained activity of the protease is at least 95% after 15 minutes of incubation at 45°C.

3. Compositions for washing, cleaning or dishwashing, containing an alkaline Bacillus protease as per one of Claims 1 or 2.

4. Compositions according to Claim 3, **characterized in that** they contain the alkaline Bacillus protease in the presence of one or more customary laundry detergent and cleaner enzymes from the group of the proteases, lipases, pectinases, amylases, nucleases, oxidoreductases and cellulases.

5. Compositions according to Claim 4, **characterized in that** they contain the alkaline Bacillus protease in the presence of a second protease.

6. Compositions according to Claim 5, **characterized in that** they contain a combination of an alkaline Bacillus protease as per Claim 1 and of an alkaline Bacillus protease as per Claim 2.

7. Compositions according to one of Claims 3 to 6, **characterized in that** they contain the alkaline Bacillus protease in a formulation for low use temperatures of up to about 60°C.

8. Process for preparing alkaline Bacillus proteases as per one of Claims 1 or 2, **characterized in that** the Bacillus pumilus DSM 5777 or a microorganism which, in an expression vector, contains the genetic information for one of the proteases indicated in Claims 1 to 2 is cultivated and the alkaline protease formed is subsequently isolated from the culture supernatant.

9. Process according to Claim 8, **characterized in that** the microorganism used is a Bacillus subtilis or a Bacillus alcalophilus or a Bacillus licheniformis or a Bacillus amyloliquefaciens or a Bacillus pumilus.

10. Process according to Claim 9, **characterized in that** the expression vector is a plasmid isolable from the microorganism deposited under the number DSM 6879 or from the microorganism deposited under the number DSM 6880.

11. Microorganisms deposited under the number DSM 6879 and the number DSM 6880.

12. Plasmid isolable from the microorganism deposited under the number DSM 6879 and containing the genetic information for the protease indicated in Claim 1.

13. Plasmid isolable from the microorganism deposited under the number DSM 6880 and containing the genetic information for the protease indicated in Claim 2.

## Revendications

1. Protéase alcaline isolée de Bacillus, pouvant être obtenue par culture de Bacillus pumilus DSM 5777, ayant les propriétés suivantes:
(1) effet: décomposition de protéines et peptides;
(2) pH optimum: à des valeurs de pH à peu près comprises entre 10,5 et 11,0
(3) stabilité avec le pH: à des valeurs de pH de 9,7 à 10,8, les enzymes se révèlent totalement stables;
(4) température optimale: environ 60°C;
(5) stabilité à la température: par incubation de la protéase à des températures jusqu'à 45°C pendant 15 minutes, la protéase n'est pas essentiellement influencée dans son activité; au bout de 15 minutes d'incubation à 50°C, l'activité résiduelle de la protéase est d'au moins 90%.

2. Protéase alcaline isolée de Bacillus, pouvant être obtenue par culture de Bacillus pumilus DSM 5777, ayant les propriétés suivantes:
(1) effet: décomposition de protéines et peptides;
(2) pH optimum: à des valeurs de pH à peu près comprises entre 8,5 et 9;
(3) stabilité avec le pH: à des valeurs de pH de 5,5 à 10,5, les enzymes se révèlent totalement stables;
(4) température optimale: environ 50°C;
(5) stabilité avec la température: par incubation de la protéase à des températures jusqu'à 40°C pendant 15 minutes, la protéase n'est pas essentiellement influencée dans son activité; au bout de 15 minutes d'incubation à 45°C, l'activité résiduelle de la protéase est d'au moins 90%.

3. Compositions pour le lavage, le nettoyage ou l'entretien de la vaisselle, contenant une protéase alcaline de Bacillus selon l'une quelconque des revendications 1 ou 2.

4. Compositions selon la revendication 3, **caractérisées en ce qu'**elles contiennent la protéase alcaline de Bacillus avec une ou plusieurs des enzymes habituelles dans des agents de lavage et de nettoyage, formées du groupe des protéases, lipases, pectinases, amylases, nucléases, oxydoréductases et cellulases.

5. Compositions selon la revendication 4, **caractérisées en ce qu'**elles contiennent la protéase alcaline de Bacillus en présence d'une seconde protéase.

6. Compositions selon la revendication 5, **caractérisées en ce qu'**elles contiennent une combinaison formée d'une protéase alcaline de Bacillus selon la revendication 1 et d'une protéase alcaline de Bacillus selon la revendication 2.

7. Compositions selon l'une quelconque des revendications 3 à 6, **caractérisées en ce qu'**elles contiennent la protéase alcaline de Bacillus dans une formulation pour de basses températures d'utilisation jusqu'à environ 60°C.

8. Procédé pour la production de protéases alcalines de Bacillus selon l'une quelconque des revendications 1 ou 2, **caractérisé en ce que** l'on cultive Bacillus pumilus DSM 5777 dans un vecteur d'expression ou un microorganisme contenant l'information génétique pour l'une des protéases indiquées aux revendications 1 ou 2, et subséquemment, on isole, du résidu de la culture, la protéase alcaline formée.

9. Procédé selon la revendication 8, **caractérisé en ce qu'**on utilise, en tant que microorganisme, Bacillus subtilis ou Bacillus alcalophilus ou Bacillus licheniformis ou Bacillus amyloliquefaciens ou Bacillus pumilus.

10. Procédé selon la revendication 9, **caractérisé en ce que** le vecteur d'expression est un plasmide pouvant être isolé du microorganisme déposé sous le numéro DSM 6879 ou d'un microorganisme déposé sous le numéro DSM 6880.

11. Microorganismes déposés sous le numéro DSM 6879 et le numéro DSM 6880.

12. Plasmide pouvant être isolé du microorganisme déposé sous le numéro DSM 6879 contenant l'information génétique pour la protéase indiquée à la revendication 1.

13. Plasmide pouvant être isolé du microorganisme déposé sous le numéro DSM 6880 contenant l'information génétique pour la protéase indiquée à la revendication 2.
